(19) Europäisches Patentamt
European Patent Office
Office européen des brevets

(11) **EP 4 502 169 A1**

(12) **EUROPEAN PATENT APPLICATION**
published in accordance with Art. 153(4) EPC

(43) Date of publication:
05.02.2025 Bulletin 2025/06

(21) Application number: 23780310.1

(22) Date of filing: 27.03.2023

(51) International Patent Classification (IPC):
*C12P 21/08* (2006.01)        *C07K 16/18* (2006.01)
*C12N 5/071* (2010.01)        *C12N 5/10* (2006.01)

(52) Cooperative Patent Classification (CPC):
C07K 16/00; C07K 16/18; C12N 5/06; C12N 5/10

(86) International application number:
PCT/JP2023/012126

(87) International publication number:
WO 2023/190300 (05.10.2023 Gazette 2023/40)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 28.03.2022 JP 2022051206

(71) Applicant: FUJIFILM Corporation
Tokyo 106-8620 (JP)

(72) Inventors:
• **NAKAI Shinichi**
  **Ashigarakami-gun, Kanagawa 258-8577 (JP)**
• **INADA Atsushi**
  **Ashigarakami-gun, Kanagawa 258-8577 (JP)**
• **SAKUYAMA Hiroshi**
  **Ashigarakami-gun, Kanagawa 258-8577 (JP)**
• **TAKAHASHI Naoto**
  **Ashigarakami-gun, Kanagawa 258-8577 (JP)**

(74) Representative: Hoffmann Eitle
**Patent- und Rechtsanwälte PartmbB
Arabellastraße 30
81925 München (DE)**

(54) **METHOD FOR PRODUCING PRODUCT AND PRODUCT**

(57) An object of the present invention is to provide a method in which the amount of mannose 5 in a product can be reduced in the production method for the product by cell culture, and a product that is produced by the method. According to the present invention, there is provided a production method for a product, including culturing cells in a culture container, in which the culturing is a perfusion culture method, Expressions (1) to (5) are satisfied in a production period of a product after reaching a target viable cell density, a variation of VCD during the production period is ±20% or less with respect to an average viable cell density during the production period, and a variation of VCV during the production period is ±20% or less with respect to an average value of the VCV during the production period.

$$0 \leq \text{Gln-C} \leq 2.5 \qquad \text{Expression (1)}$$

$$0.5 \leq \text{Gluc-C} \leq 8.0 \qquad \text{Expression (2)}$$

$$1.0 \leq \text{NH4-C} \leq 8.0 \qquad \text{Expression (3)}$$

$$50 \leq \text{VCD} \leq 300 \qquad \text{Expression (4)}$$

$$0.6 \times 10^5 \leq \text{VCV} \leq 4.0 \times 10^5 \qquad \text{Expression (5)}$$

EP 4 502 169 A1

The meanings of the symbols in the expressions are as defined in the present specification.

## FIG. 1

**Description**

Technical Field

The present invention relates to a production method for a product, which includes culturing cells under predetermined conditions in a production period of a product after reaching a target viable cell density. The present invention further relates to a product that is produced by the above-described production method.

Background Art

Cell culture is carried out for the intended purpose, for example, increasing the number of cells having useful properties or causing cells to produce a product.

For example, Patent Document 1 discloses a production method for a double-specific antibody product, the method including (i) a step of supplying at least one kind of mammalian cell capable of expressing a double-specific antibody product in a perfusion bioreactor, (ii) a step of proliferating a mammalian cell culture at a first perfusion amount until a set value of a viable cell density is reached, and (iii) a step of maintaining perfusion culture at a second perfusion amount where the step is such that the concentration of the double-specific antibody product in the bioreactor is maintained to be lower than a threshold value.

Patent Document 2 discloses an integrated continuous method for producing a therapeutic protein bulk drug, the method including (i) supplying a liquid culture medium containing a recombinant therapeutic protein but substantially not containing a cell so that the liquid culture medium is fed into a first multi-column chromatography system (MCCS1); (ii) capturing the recombinant therapeutic protein in the liquid culture medium by using the MCCS1, and continuously feeding an eluate of the MCCS1 containing the recombinant therapeutic protein to a second multi-column chromatography system (MCCS2); and (iii) purifying and polishing the recombinant therapeutic protein by using the MCCS2 so that the eluate eluted from the MCCS2 is the therapeutic protein bulk drug.

Patent Document 3 discloses a method of controlling a perfusion rate of a perfusion bioreactor, the method including a) measuring a viable cell density of cells that proliferate in culture by using a biomass electrostatic capacity probe and b) dynamically adjusting a target perfusion rate in consideration of the viable cell density, in which the cells proliferate to a high cell density during perfusion.

Prior Art Documents

Patent Documents

    Patent Document 1: JP2021-505182A
    Patent Document 2: JP2020-033364A
    Patent Document 3: JP2020-533983A

**SUMMARY OF THE INVENTION**

Object to be solved by the invention

Among the antibodies produced by cell culture, an antibody in which the sugar chain moiety has been subjected to a sugar chain modification with five mannoses (a mannose 5-modified antibody) has insufficient drug efficacy. Therefore, there is a demand for reducing the amount of the mannose 5 from the viewpoint of enhancing the drug efficacy of the pharmaceutical drug. On the other hand, it is difficult to separate and remove the mannose 5-modified antibody in the purification step, and it is desirable to suppress the production of the mannose 5-modified antibody by cells in the culture step. An object to be achieved by the present invention is to provide a method that makes it possible to reduce the amount of the mannose 5-modified antibody in the product in a production method for a product by cell culture. Further, another object of the present invention is to provide a product that is produced by the above-described method.

Means for solving the object

As a result of diligent studies to achieve the above objects, the inventors of the present invention found that the above-described object can be achieved by culturing cells under predetermined conditions in a production period of a product after reaching a target viable cell density, whereby the present invention was completed.

That is, according to the present invention, the following inventions are provided.

<1> A production method for a product, comprising:

culturing cells in a culture container,
in which the culturing is a perfusion culture method,
Expressions (1) to (5) are satisfied in a production period of a product after reaching a target viable cell density,
a variation of VCD during the production period is ±20% or less with respect to an average viable cell density during the production period, and
a variation of VCV during the production period is ±20% or less with respect to an average value of the VCV during the production period,

$$0 \leq \text{Gln-C} \leq 2.5 \qquad \text{Expression (1)}$$

$$0.5 \leq \text{Gluc-C} \leq 8.0 \qquad \text{Expression (2)}$$

$$1.0 \leq \text{NH4-C} \leq 8.0 \qquad \text{Expression (3)}$$

$$50 \leq \text{VCD} \leq 300 \qquad \text{Expression (4)}$$

$$0.6 \times 10^5 \leq \text{VCV} \leq 4.0 \times 10^5 \qquad \text{Expression (5)}$$

in the expressions, Gln-C indicates a glutamine concentration [mmol/L] in a culture solution, Gluc-C indicates a glucose concentration [g/L] in the culture solution, NH4-C indicates an ammonia concentration [mmol/L] in the culture solution, VCD indicates a viable cell density [$\times 10^6$ cells/mL], VCV indicates VCD $\times \pi/6 \times$ Cd$^3$ [$\times 10^6$ $\mu$m$^3$/mL], and Cd indicates an average diameter [$\mu$m] of viable cells.

<2> The production method for a product according to <1>, in which the culture solution is bled such that the variation of VCD during the production period is ±20% or less with respect to the average viable cell density during the production period.
<3> The production method for a product according to <2>, in which in the bleeding, the culture solution is bled while an electrostatic capacity of the culture solution is subjected to in-line measurement.
<4> The production method for a product according to <3>, in which the bleeding is automatically controlled.
<5> The production method for a product according to <3> or <4>, in which a variation of the electrostatic capacity of the culture solution, where the electrostatic capacity is subjected to in-line measurement, is ±20% or less with respect to an average value of an electrostatic capacity during the production period.
<6> The production method for a product according to any one of <3> to <5>, in which the bleeding includes:

(i) setting an electrostatic capacity serving as a target;
(ii) setting a weight control value of the culture container;
(iii) measuring the electrostatic capacity of the culture solution at a period of 0.1 seconds or less;
(iv) driving a pump for draining the culture solution at a rate of 0.3 vvd to 3 vvd in a case where the electrostatic capacity of the culture solution exceeds the target (here, vvd means "volume of drained culture solution/volume of culture solution/day"); and
(v) automatically supplying a culture medium to the culture container while measuring a mass of the culture solution such that a variation of a liquid amount of the culture solution is within 10%; and
(vi) stopping the pump for draining the culture solution in a case where the electrostatic capacity of the culture solution is smaller than the target by 0.01% or more.

<7> The production method for a product according to any one of <1> to <6>, in which Expression (6) is satisfied in the production period,

$$2 \leq \text{LDH/VCD} \leq 40 \qquad \text{Expression (6)}$$

in the expression, LDH indicates a concentration [U/L] of lactate dehydrogenase in the culture solution, and VCD indicates a viable cell density [$\times 10^6$ cells/mL],

<8> The production method for a product according to any one of <1> to <7>, in which the cell is an animal cell.

<9> The production method for a product according to any one of <1> to <8>, in which the cell is a cell that produces a protein.

<10> The production method for a product according to any one of <1> to <9>, in which the cell is a cell that produces an antibody.

<11> The production method for a product according to any one of <1> to <10>, in which the cell is a CHO cell.

<12> A product that is produced by the production method for a product according to any one of <1> to <11>.

Effect of the invention

According to the present invention, the amount of mannose 5 in the product can be reduced in a production method for a product by cell culture.

**BRIEF DESCRIPTION OF THE DRAWINGS**

Fig. 1 shows a cell culture device.

Fig. 2 shows results obtained by measuring a transition of VCV in Example 1.

Fig. 3 shows results obtained by measuring a transition of electrostatic capacity data (also denoted as Cp) in Example 1.

Fig. 4 shows results obtained by measuring a transition of a mannose 5 ratio in Example 1.

Embodiments for carrying out the invention

Hereinafter, the contents of the present invention will be described in detail. In the present specification, a numerical value range indicated using "to" means a range including numerical values described before and after "to" as a minimum value and a maximum value, respectively.

A production method for a product according to an embodiment of the present invention is a method including culturing cells in a culture container, in which the culturing is a perfusion culture method, Expressions (1) to (5) are satisfied in a production period of a product after reaching a target viable cell density, a variation of VCD during the production period is $\pm 20\%$ or less with respect to an average viable cell density during the production period, and a variation of VCV during the production period is $\pm 20\%$ or less with respect to an average value of the VCV during the production period. Since the production method for a product according to the embodiment of the present invention satisfies the above-described conditions, it is possible to reduce an amount of a mannose 5-modified antibody in the product.

$$0 \leq \text{Gln-C} \leq 2.5 \qquad\qquad \text{Expression (1)}$$

$$0.5 \leq \text{Gluc-C} \leq 8.0 \qquad\qquad \text{Expression (2)}$$

$$1.0 \leq \text{NH4-C} \leq 8.0 \qquad\qquad \text{Expression (3)}$$

$$50 \leq \text{VCD} \leq 300 \qquad\qquad \text{Expression (4)}$$

$$0.6 \times 10^5 \leq \text{VCV} \leq 4.0 \times 10^5 \qquad\qquad \text{Expression (5)}$$

In the expression,

Gln-C indicates a glutamine concentration [mmol/L] in a culture solution,

Gluc-C indicates a glucose concentration [g/L] in the culture solution,

NH4-C indicates an ammonia concentration [mmol/L] in the culture solution,

VCD indicates a viable cell density [$\times 10^6$ cells/mL],

VCV indicates VCD $\times \pi/6 \times \text{Cd}^3$ [$\times 10^6$ $\mu\text{m}^3$/mL], and

Cd indicates an average diameter [μm] of viable cells.

A preferred aspect of Expression (1) is Expressions (1A) and (1B) below.

$$0.1 \leq \text{Gln-C} \leq 2.0 \qquad \text{Expression (1A)}$$

$$0.2 \leq \text{Gln-C} \leq 1.5 \qquad \text{Expression (1B)}$$

A preferred aspect of Expression (2) is Expressions (2A) and (2B) below.

$$1.0 \leq \text{Gluc-C} \leq 6.0 \qquad \text{Expression (2A)}$$

$$1.5 \leq \text{Gluc-C} \leq 5.0 \qquad \text{Expression (2B)}$$

A preferred aspect of Expression (3) is Expressions (3A) and (3B) below.

$$1.5 \leq \text{NH4-C} \leq 7.0 \qquad \text{Expression (3A)}$$

$$2.0 \leq \text{NH4-C} \leq 6.0 \qquad \text{Expression (3B)}$$

A preferred aspect of Expression (4) is Expressions (4A) and (4B) below.

$$80 \leq \text{VCD} \leq 250 \qquad \text{Expression (4A)}$$

$$100 \leq \text{VCD} \leq 220 \qquad \text{Expression (4B)}$$

A preferred aspect of Expression (5) is Expressions (5A) and (5B) below.

$$0.8 \times 10^5 \leq \text{VCV} \leq 3.0 \times 10^5 \qquad \text{Expression (5A)}$$

$$1.0 \times 10^5 \leq \text{VCV} \leq 2.5 \times 10^5 \qquad \text{Expression (5B)}$$

The variation of the VCD during the production period is ±20% or less with respect to the average viable cell density during the production period. However, it is more preferable that the variation of the VCD during the production period is ±15% or less with respect to an average viable cell density during the production period, it is still more preferable that the variation of the VCD during the production period is ±12% or less with respect to an average viable cell density during the production period, and it is particularly preferable that the variation of the VCD during the production period is ±8% or less with respect to an average viable cell density during the production period. The lower limit of the variation of the VCD during the production period is not particularly limited, and it is sufficient to be 0% or more; however, a variation of substantially 0.1% or more occurs in consideration of the variation of daily cell proliferation.

The variation of the VCV during the production period is ±20% or less with respect to the average value of the VCV during the production period. However, it is more preferably ±15% or less with respect to the average value of the VCV during the production period, still more preferably ±12% or less with respect to the average value of the VCV during the production period, and particularly preferably ±8% or less with respect to the average value of the VCV during the production period. The lower limit of the variation of the VCV during the production period is not particularly limited, and it is sufficient to be 0% or more; however, a variation of substantially 0.1% or more occurs in consideration of the variation of daily cell proliferation.

The glutamine concentration [mmol/L] in a culture solution, which is represented by Gln-C, can be measured using a filtrate obtained by extracting the culture solution during culture from a culture container and then removing cells by filtration. The glutamine concentration in the filtrate can be measured using a commercially available analysis apparatus such as Bioprofile FLEX2 manufactured by Nova Biomedical K.K. The frequency of the measurement of the glutamine concentration is not particularly limited; however, it can be carried out, for example, once a day.

The glucose concentration [g/L] in a culture solution, which is represented by Gluc-C, can be measured using a filtrate obtained by extracting the culture solution during culture from a culture container and then removing cells by filtration. The glucose concentration in the filtrate can be measured using a commercially available analysis apparatus such as Bioprofile FLEX2 manufactured by Nova Biomedical K.K. The frequency of the measurement of the glucose concentration is not particularly limited; however, it can be carried out, for example, once a day.

The ammonia concentration [mmol/L] in a culture solution, which is represented by NH4-C, can be measured using a filtrate obtained by extracting the culture solution during culture from a culture container and then removing cells by filtration. The ammonia concentration in the filtrate can be measured using a commercially available analysis apparatus such as Bioprofile FLEX2 manufactured by Nova Biomedical K.K. The frequency of the measurement of the ammonia concentration is not particularly limited; however, it can be carried out, for example, once a day.

The viable cell density [$\times 10^6$ cells/mL] indicated by VCD can be measured by extracting the culture solution during culture from the culture container and subjecting it to a measurement according to a conventional method. In the present invention, VCD $\times \pi/6 \times Cd^3$ [$\times 10^6 \mu m^3$/mL] is further calculated as the VCV Here, Cd indicates the average diameter [$\mu m$] of viable cells.

The viable cell density and the average diameter of the viable cells can be measured using a commercially available measuring device such as Cell Viability Analyzer Vi-cell XR manufactured by Beckman Coulter, Inc., and as an example, they can be measured by using Vi-cell XR 2.04 as the Vi-cell software and setting the Min diameter to 6 $\mu m$ and the Max diameter to 50 $\mu m$ as parameters at the time of measurement. In addition, the measurement parameters can be set as follows.

Cell brightness: 85%
Cell sharpness: 100
Viable cell spot brightness: 75%
Viable cell spot area: 5%
Minimum circularity: 0
Decluster degree: Medium

It is noted that the sample at the time of measurement may be used without dilution, or a sample after dilution may be used. The frequency of the measurements of the viable cell density and the average diameter of the viable cells is not particularly limited; however, they can be carried out, for example, once a day.

The variation of the VCD during the production period is a variation rate (percentage) that indicates how much the VCD value measured during the production period is varied with respect to the average value of the VCD during the production period, that is, the average viable cell density.

The average value of the VCD during the production period can be determined by extracting the culture solution in the culture tank every day during the production period, adding a value of the VCD measured using Cell Viability Analyzer Vi-cell XR or the like manufactured by Beckman Coulter, Inc. for the number of days of culture during the production period, and dividing the sum by the number of days of culture during the production period.

The variation of the VCV during the production period is a variation rate (percentage) that indicates how much the VCV value measured during the production period is varied with respect to the average value of the VCV during the production period.

The average value of the VCV during the production period can be determined by extracting the culture solution in the culture tank every day during the production period, calculating VCV [$\times 10^6 \mu m^3$/ml] according to the expression of VCV = VCD $\times (\pi/6 \times Cd^3)$ from the VCD measured using Cell Viability Analyzer Vi-cell XR or the like manufactured by Beckman Coulter, Inc. and a value of the average diameter Cd of cells, adding a value of the VCV for the number of days of culture during the production period, and dividing the sum by the number of days of culture during the production period.

In the present invention, preferably, the culture solution can be bled such that the variation of VCD during the production period is $\pm 20\%$ or less with respect to an average viable cell density during the production period. The bleeding means that a part of the culture solution is extracted together with cells so that the viable cell density during culture is not excessive, whereby the viable cell density is reduced. In order to maintain the amount of the culture solution, it is possible to add an amount of a fresh culture medium, which is equivalent to the amount of the extracted culture solution.

It is more preferable that the variation of the VCD during the production period is $\pm 15\%$ or less with respect to an average viable cell density during the production period, it is still more preferable that the variation of the VCD during the production period is $\pm 12\%$ or less with respect to an average viable cell density during the production period, and it is particularly preferable that the variation of the VCD during the production period is $\pm 8\%$ or less with respect to an average viable cell density during the production period. The lower limit of the variation of the VCD during the production period is not particularly limited, and it is sufficient to be 0% or more; however, a variation of substantially 0.1% or more occurs in consideration of the variation of daily cell proliferation.

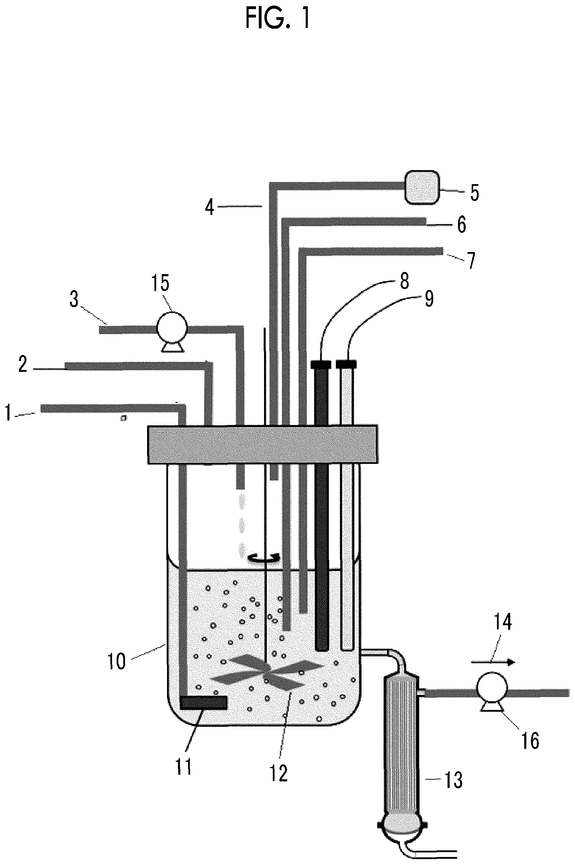

The bleeding is sufficient to be such that the culture solution containing cells can be extracted from the culture container, and the method thereof is not particularly limited. However, for example, as shown in Fig. 1, a pipe for discharging the culture solution is inserted into the culture solution, and the culture solution can be discharged from the culture container through the pipe.

Preferably, in the bleeding, the culture solution may be bled while an electrostatic capacity of the culture solution is subjected to in-line measurement. The in-line measurement is to measure any measurement value indicating a state of the culture solution while carrying out cell culture. In order to subject the electrostatic capacity to in-line measurement, for example, as shown in Fig. 1, it is sufficient to insert an electrostatic capacity sensor into the culture solution and subject the electrostatic capacity of the culture solution to in-line measurement while carrying out culture.

Preferably, the bleeding may be automatically controlled. The automatic control means that the start or stop of the bleeding is automatically controlled based on any measurement value indicating any state of the culture solution. For example, the start and stop of the bleeding can be automatically controlled based on the measurement value of the electrostatic capacity of the culture solution.

Preferably, the bleeding may be carried out such that a variation of the electrostatic capacity of the culture solution, where the electrostatic capacity is subjected to in-line measurement, is $\pm20\%$ or less with respect to an average value of an electrostatic capacity during the production period. The variation of the electrostatic capacity of the culture solution, where the electrostatic capacity is subjected to in-line measurement, is more preferably $\pm15\%$ or less with respect to an average value of an electrostatic capacity during the production period, still more preferably $\pm12\%$ or less with respect to the average value of the electrostatic capacity during the production period, and particularly preferably $\pm8\%$ or less with respect to the average value of the electrostatic capacity during the production period. The lower limit of the variation of the electrostatic capacity of the culture solution is not particularly limited, and it is sufficient to be 0% or more; however, a variation of substantially 0.1% or more occurs in consideration of the variation of daily cell proliferation.

One exemplary bleeding can be carried out by a step including:

(i) setting an electrostatic capacity serving as a target;
(ii) setting a weight control value of the culture container;
(iii) measuring the electrostatic capacity of the culture solution at a period of 0.1 seconds or less;
(iv) driving a pump for draining the culture solution at a rate of 0.3 vvd to 3 vvd in a case where the electrostatic capacity of the culture solution exceeds the target (here, vvd means "volume of drained culture solution/volume of culture solution/day");
(v) automatically supplying a culture medium to the culture container while measuring a mass of the culture solution such that a variation of a liquid amount of the culture solution is within 10%; and
(vi) stopping the pump for draining the culture solution in a case where the electrostatic capacity of the culture solution is smaller than the target by 0.01% or more.

In the present invention, it is preferable that Expression (6) is satisfied in the production period.

$$2 \leq \mathrm{LDH/VCD} \leq 40 \qquad \text{Expression (6)}$$

In the expression, LDH indicates a concentration [U/L] of the lactate dehydrogenase in the culture solution, and VCD indicates a viable cell density [$\times 10^6$ cells/mL],

A more preferred aspect of Expression (6) is Expressions (6A) and (6B) below.

$$2 \leq \mathrm{LDH/VCD} \leq 35 \qquad \text{Expression (6A)}$$

$$2 \leq \mathrm{LDH/VCD} \leq 30 \qquad \text{Expression (6B)}$$

The concentration [U/L] of the lactate dehydrogenase in a culture solution, which is represented by LDH - C, can be measured using a filtrate obtained by extracting the culture solution during culture from a culture container and then removing cells by filtration. The concentration of the lactate dehydrogenase in the filtrate can be measured using a commercially available analysis apparatus such as Cedex Bio manufactured by F. Hoffmann-La Roche, Ltd. The frequency of the measurement of the concentration of the lactate dehydrogenase is not particularly limited; however, it can be carried out, for example, once a day.

Fig. 1 shows one example of a cell culture device that can be used in the cell culture in the present invention. In Fig. 1, a culture container 10 is a container that accommodates a culture solution containing cells. Cells are cultured in the culture solution in the inside of the culture container 10.

Oxygen and air are sent from a sparger air supply pipe 1, and the oxygen and the air are introduced into the culture solution through a sparger 11 having a hole diameter of 20 $\mu$m. The dissolved oxygen concentration inside the culture solution can be adjusted by the sparger 11. The sparger is not particularly limited; however, it is possible to use, for example, a sparger in which an average hole diameter of a gas release unit is 1 $\mu$m or more and 300 $\mu$m or less and which releases a gas containing 30% by volume or more of oxygen.

Air and carbon dioxide are introduced from an air supply pipe 2 to an upper part of the culture solution in the culture container.

The culture medium is supplied to the culture container from a culture medium supply pipe 3. A culture medium supply pump 15 is provided in the culture medium supply pipe 3.

A gas exhaust pipe 4 is a pipe for gas exhaust, and a gas exhaust filter 5 is connected to a terminal of the gas exhaust pipe 4.

A sampling tube 6 is a pipe for collecting (sampling) the culture solution.

A bleeding tube 7 is a pipe for extracting (bleeding) the culture solution.

An electrostatic capacity sensor 8 is mounted to come into contact with the culture solution in the culture container.

A dissolved oxygen sensor 9 is mounted to come into contact with the culture solution in the culture container.

A stirring member having a stirring blade 12 may be provided inside the culture container 10. In a case where the stirring blade 12 is rotated, the culture solution inside the culture container 10 is stirred, and the homogeneity of the culture solution is maintained. In a case where the culture solution is stirred by the stirring blade 12, the bubbles released by the sparger are also stirred. The position of the stirring member having a stirring blade, the size of the stirring blade, and the like are not particularly limited and may be designed depending on the cell kind to be used, the amount of the culture solution, the amount of oxygen to be supplied, or the position, number, size, or the like of the sparger. Further, in order to quickly stir bubbles coming out of the sparger and suppress coalescence of the bubbles, it is preferable to dispose the stirring blade 12 at a position close to the sparger.

A perfusion device 13 is connected to a lower part of the culture container 10. In addition, the culture solution inside the culture container 10 passes through the perfusion device 13, and the permeated solution containing the cell product is extracted from the culture container 10 by a delivery pump 16. The flow of the permeated solution is indicated by an arrow 14.

In the present invention, The viable cell density of the culture solution is preferably $50 \times 10^6$ to $300 \times 10^6$ cells/mL, more preferably $80 \times 10^6$ to $250 \times 10^6$ cells/mL, and still more preferably $100 \times 10^6$ to $220 \times 10^6$ cells/mL. M may be used to denote $10^6$.

In the present invention, a cell suspension extracted from the culture container may be allowed to pass through a separation membrane to separate the cell suspension into a cell-containing liquid and a permeated solution. This operation can be carried out using a perfusion device. In this operation, the cell suspension extracted from the culture container is separated into a cell-containing liquid having a cell density higher than that of the cell suspension and a permeated solution having a cell density lower than that of the cell suspension. The cell density can be measured by a live/dead cell analyzer Vi-CELL XR, manufactured by Beckman Coulter Life Sciences.

The membrane separation treatment step described above is preferably tangential filtration, more preferably alternating tangential flow (ATF) or tangential flow, and most preferably alternating tangential flow. Examples of the filter capable of carrying out the alternating tangential flow include SuATF10-S02PES or F2 RF02PES, manufactured by Repligen Corporation.

As the culture medium that is used for cell culture, a culture medium that is generally used for culturing animal cells can be used. For example, CD OptiCHO (manufactured by Thermo Fisher Scientific, Inc.), Dulbecco's modified Eagle medium (DMEM), Eagle minimum essential medium (MEM), RPMI-1640 medium, RPMI-1641 medium, F-12K medium, Ham's F12 medium, Iscove's modified Dulbecco's medium (IMDM), McCoy's 5A medium, Leibovitz's L-15 medium, and EX-CELL (trade mark) 300 series (JRH Biosciences), CHO-S-SFMII (Invitrogen), CHO-SF (Sigma-Aldrich Co. LLC), CD-CHO (Invitrogen), ISCHO-V (FUJIFILM Irvine Scientific), PF-ACF-CHO (Sigma-Aldrich Co. LLC), and the like can be used. Alternatively, a homemade culture medium may be used.

Serum such as fetal calf serum (FCS) may be added to the culture medium, or serum may not be added thereto. The culture medium may be supplemented with additional components such as an amino acid, salts, a sugar, a vitamin, a hormone, a growth factor, a buffer solution, an antibiotic, a lipid, a trace element, and a hydrolysate of a plant protein. A protein-free culture medium can also be used.

Although the pH of the culture medium varies depending on the cells to be cultured, the culture medium generally has a pH of 6.0 to 8.0, preferably has a pH of 6.4 to 7.6, and more preferably has a pH of 6.7 to 7.4.

The culture temperature is generally 30°C to 40°C, preferably 32°C to 39°C, and more preferably 36°C to 38°C, and the culture temperature may be changed during the culture.

The culture can be carried out in an atmosphere having a $CO_2$ concentration of 0% to 40% by volume, preferably 2 to 25% by volume, and more preferably 3 to 20% by volume.

The amount of the culture solution is preferably 1 L or more, more preferably 50 L or more, and still more preferably 200 L or more.

The culture period is not particularly limited; however, it is generally 12 hours to 90 days, preferably 1 day to 80 days, more preferably 1 day or more and less than 70 days, still more preferably 5 days or more and less than 65 days, and particularly preferably 7 days or more and less than 60 days. In the present invention, the culture period is preferably 14 days or more.

In the culture, the culture medium can be replaced, aerated, and stirred as necessary. In a case of carrying out stirring of the culture, the rotation speed of stirring is not particularly limited; however, the stirring power per unit volume is generally 10 to 300 kW/m$^3$, preferably 20 to 200 kW/m$^3$, and more preferably 30 to 100 kW/m$^3$.

The dissolved oxygen concentration in the culture solution can be appropriately set and is not particularly limited; however, it is 20% to 150%, preferably 30% to 120%, and more preferably 50% to 100% in a case where the saturated dissolved oxygen concentration in the liquid at 37°C in air at 1 atm is set to 100%.

The cell culture can be carried out using a cell culture device having the configuration described above in the present specification. The cell culture device may be any one of a fermenter tank type culture device, an air lift type culture device, a culture flask type culture device, a spinner flask type culture device, a microcarrier type culture device, a fluidized bed type culture device, a hollow fiber type culture device, a roller bottle type culture device, a filling tank type culture device, or the like. The culture container is preferably a single-use culture tank from the viewpoint of homogenizing the culture environment or the like. From the viewpoint of homogenization of the culture environment and the like, the cell culture device may be a single-use culture tank.

The viscosity of the culture solution is preferably 1.2 mPa·s or more and less than 15 mPa s, more preferably 1.4 mPa s or more and less than 12 mPa s, and particularly preferably 1.6 mPa·s or more and less than 10 mPa·s.

In the present invention, a pH adjusting agent may be added during the culture. From the viewpoint of the clogging of the filtration filter flow channel, a pH adjusting agent to be added to the culture tank is such that an adding amount thereof per day is preferably 8 mmol/day/L or less and more preferably 7 mmol/day/L or less. Here, it is not necessary to add a pH adjusting agent.

The pH adjusting agent is not particularly limited; however, it is preferably an aqueous Na$_2$CO$_3$ solution, an aqueous NaOH solution, or an aqueous NaHCO$_3$ solution, and it is more preferably an aqueous NaHCO$_3$ solution. The pH adjusting agent may be added alone or may be added by being mixed with a culture medium or an anti-foaming agent. In order to avoid the local variation of pH in the culture solution due to the addition of the pH adjusting agent, it is preferable that the pH adjusting agent is added by being mixed with a culture medium.

In the present invention, the mode of the method of culturing cells is perfusion culture.

The perfusion culture is a culture method in which a fresh culture medium is added and at the same time the used culture medium is removed. In general, the perfusion culture makes it possible to achieve a high viable cell density. A typical perfusion culture begins with a batch culture start-up lasting 1 or 2 days, thereafter a fresh supply culture medium is added to the culture continuously, stepwise, and/or intermittently, and the used culture medium is removed at the same time. In the perfusion culture, methods such as sedimentation, centrifugation, and filtration can be used to remove the used culture medium while maintaining the viable cell density. The advantage of the perfusion culture is that the culture in which a target protein is produced is maintained for a long period as compared with the batch culture method or the fed-batch culture.

Perfusion may be any form of being continuous, stepwise, intermittent, or a combination thereof. A continuous form is preferable. Animal cells are retained in the culture and the used culture medium that is removed may substantially not include cells or may have much fewer cells than the culture. A product that is expressed by cell culture can be retained in the culture or harvested by the selection of the membrane hole diameter.

The number of days of the perfusion culture is preferably 5 days or more and 150 days or less, more preferably 10 days or more and 100 days or less, and still more preferably 20 days or more and 90 days or less. It is preferable for the number of days of the culture to be 5 days or more since the obtained cell number is large and the production amount of a product is large in a case where the cells produce the product. It is preferable for the number of days of the culture to be 150 days or less from the viewpoint of preventing clogging of the filtration membrane used in the perfusion culture and preventing contamination.

The perfusion ratio is not particularly limited; however, it is generally 0.3 vvd to 5.0 vvd, preferably 0.5 vvd to 3.0 vvd, and more preferably 0.8 vvd to 2.5 vvd. The vvd regarding the perfusion ratio means "volume of drained culture solution/volume of culture solution in culture container/day". It is noted that the perfusion ratio described herein is a parameter different from "(iv) driving a pump for draining the culture solution at a rate of 0.3 vvd to 3 vvd in a case where the electrostatic capacity of the culture solution exceeds the target" in one example of the bleeding.

The extraction of the culture solution from the culture container can usually be carried out using a pump, but other available liquid feeding units may be used. The culture solution extracted from the culture container is subjected to treatments such as product collection and removal of the dead cells. The culture solution extracted from the culture container may be partially discarded or returned to the culture container after treatments such as product collection and removal of the dead cells. In a case where a loss of the culture medium occurs due to the above treatments, the loss can be compensated, for example, by supplying a fresh culture medium to the culture container.

The kind of the cell in the present invention is not particularly limited; however, examples thereof include eukaryotic cells such as an animal cell, a plant cell, and yeast, prokaryotic cells such as Bacillus subtilis, and Escherichia coli. The cell is preferably an animal cell (more preferably a mammalian cell) or an insect cell, and it is most preferably a mammalian cell. The cell may be a primary cell or a cell established as a cell line.

Examples of the cell include a Chinese hamster ovary (CHO) cell, a HEK cell (a cell derived from the human embryonic kidney), a BHK cell, a 293 cell, a C127 cell, a myeloma cell (such as an NS0 cell), a PerC6 cell, a SP2/0 cell, a hybridoma cell, a COS cell (a cell derived from the kidney of the African green monkey), a 3T3 cell, a HeLa cell, a Vero cell (a renal epithelial cell of the African green monkey), a MDCK cell (a cell derived from a canine kidney renal tubular epithelial cell), a PC12 cell, and a WI38 cell. The cell may be a stem cell such as an embryonic stem cell (ES cell) or an induced pluripotent stem cell (iPS cell). Among these, a CHO cell, a HEK cell, a BHK cell, or a hybridoma is preferable, where a CHO cell or a HEK cell is more preferable, and a CHO cell is most preferable. The CHO cell is widely used for the production of recombinant proteins such as a cytokine, a coagulation factor, and an antibody. It is preferable to use a CHO cell deficient in dihydrofolate reductase (DHFR), and as a DHFR-deficient CHO cell, it is possible to use, for example, CHO-DG44.

It is preferable that the cell viability is high; however, it is preferably 80% or more, more preferably 85% or more, particularly preferably 90% or more, and most preferably 95% or more.

These cells may be cells into which a foreign gene encoding a protein desired to be expressed (for example, an antibody) has been introduced. The cell is preferably a cell that produces an antibody. An expression vector can be used for introducing a foreign gene encoding a protein desired to be expressed, into a cell. An expression vector containing a DNA encoding a protein desired to be expressed, an expression control sequence (for example, an enhancer, a promoter, a terminator, or the like), and a selection marker gene as desired is introduced into a cell, whereby it is possible to prepare a cell into which a foreign gene encoding a protein desired to be expressed is introduced. The expression vector is not particularly limited and can be appropriately selected and used depending on the kind, use application, and the like of the cell.

As the promoter, it is possible to use any promoter of which the function can be exhibited in mammalian cells. Examples thereof include a promoter of the immediate early (IE) gene of the cytomegalovirus (CMV), an early promoter of SV40, a retrovirus promoter, a metallothionein promoter, a heat shock promoter, an $SR\alpha$ promoter, and a promoter and enhancer of the Moloney murine leukemia virus. In addition, an enhancer of the IE gene of human CMV may be used together with the promoter.

As the selection marker gene, it is possible to use, for example, a drug resistance gene (a neomycin resistance gene, a DHFR gene, a puromycin resistance gene, a blasticidin resistance gene, a hygromycin resistance gene, a cycloheximide resistance gene), or a fluorescence gene (a gene encoding a green fluorescent protein GFP or the like).

The method of introducing an expression vector into a cell is not particularly limited, and it is possible to use, for example, a calcium phosphate method, an electroporation method, a liposome method, a gene gun method, or a lipofection method.

The method for producing a product according to the embodiment of the present invention includes culturing a cell using a cell culture device to produce a product from the cell.

According to the present invention, there is provided a product that is produced by the product production method for a product according to the embodiment of the present invention.

In the present invention, the kind of product is not particularly limited; however, the product is preferably a recombinant protein. Examples of the product include a recombinant polypeptide chain, a recombinant secreted polypeptide chain, an antigen-binding protein, an antibody (for example, a human antibody, a humanized antibody, a chimeric antibody, a mouse antibody, or a bispecific antibody), an Fc fusion protein, a fragmented immunoglobulin, and a single-chain antibody (scFv). In addition, it may be, in addition to the above, an adenovirus, an adeno-associated virus, a lentivirus, or the like.

The product is preferably an antibody, and more preferably a human antibody, a humanized antibody, a chimeric antibody, or a mouse antibody. Examples of the fragmented immunoglobulin include Fab, F(ab')2, and Fv. The class of the antibody is also not particularly limited, and it may be any class of IgG such as IgG1, IgG2, IgG3, or IgG4, IgA, IgD, IgE, or IgM. However, IgG or IgM is preferable in a case of being used as a medicine.

The human antibody includes all antibodies having one or a plurality of variable and constant regions induced from human immunoglobulin sequences. In one embodiment, all variable and constant domains are induced from human immunoglobulin sequences (complete human antibodies).

In a case of being administered to a human subject, the humanized antibody has a sequence different from a sequence of an antibody induced from a non-human species by substitution, deletion, and/or addition of one or a plurality of amino acids so that there is a low possibility for the humanized antibody to induce an immune response and/or so that induction of a severe immune response is reduced as compared with the antibody of non-human species. In one example, specific amino acids in a framework and constant domains of heavy chains and/or light chains of an antibody of non-human species are mutated to produce a humanized antibody. In another example, a constant domain from a human antibody is fused to a variable domain of an antibody of a non-human species.

The chimeric antibody is an antibody in which variable regions and constant regions having origins different from each other are linked. For example, an antibody consisting of variable regions of heavy chains and light chains of a mouse antibody and consisting of constant regions of heavy chains and light chains of a human antibody is a mouse/human heterologous chimeric antibody. It is possible to prepare a recombinant vector expressing a chimeric antibody by linking a DNA encoding variable regions of a mouse antibody and a DNA encoding constant regions of a human antibody and then incorporating the linked DNA into an expression vector. It is possible to acquire a chimeric antibody produced during the culture by culturing a recombinant cell transformed with the above vector and expressing the incorporated DNA.

The bispecific antibody is an antibody that recognizes two kinds of antigenic specificity, which are different from each other. Various forms of bispecific antibodies are present. As a method of preparing a bispecific antibody, it has been reported a method of preparing a bispecific antibody by binding two immunoglobulin molecules by using a crosslinking agent such as N-succinimidyl 3-(2-pyridyldithiol)propionate or S-acetylmercaptosuccinic acid anhydride, a method of preparing a bispecific antibody by binding Fab fragments of immunoglobulin molecules to each other. In addition, a bispecific antibody can be expressed by introducing a gene encoding the bispecific antibody into a cell.

The Fc fusion protein indicates a protein having an Fc region and includes an antibody.

The Fab is a monovalent fragment having VL, VH, CL, and CH1 domains.

The F(ab')2 is a divalent fragment having two Fab fragments bound by a disulfide crosslinking at a hinge region.

The Fv fragment has VL and VH domains of a single arm of an antibody.

The single-chain antibody (scFv) is an antibody in which VL and VH regions are joined through a linker (for example, a synthetic sequence of amino acid residues) to form a continuous protein chain, where the linker is long enough to allow the protein chain to fold for itself and form a monovalent antigen binding site.

The antibody is not particularly limited; however, examples thereof include an anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, and an anti-VLA4 antibody.

Regarding the product collection, the culture solution may be simply collected. For example, a liquid obtained by removing at least a part of the cells from the culture solution using a filter or a centrifuge may be collected, and a known method is used without particular limitation. In a case of desiring to improve the purity of the product, change the solvent of the product, or change the form of the product to, for example, a power form, the culture solution or the liquid can be subjected to further treatment.

In addition, a part of the culture solution can be collected while being perfused, or a part of the culture solution can be collected as a liquid which is obtained by removing at least a part of cells from the part of the culture solution, which is filtered or centrifuged while being perfused.

The product can be purified by a purification treatment. The obtained product can be purified to high purity. For the separation and purification of the product, the separation and purification methods that are used for general proteins may be used. For example, it is possible to carry out separation and purification of a product by appropriately selecting and combining means such as a chromatography such as affinity chromatography, a filter, ultrafiltration, salting out, dialysis, sodium dodecyl sulfate (SDS) polyacrylamide gel electrophoresis, isoelectric focusing electrophoresis, and the like, which are not limited thereto. The concentration of the product obtained as above can be measured according to absorbance measurement, enzyme-linked immunosorbent assay (ELISA), or the like. In addition, in a case where the product is an antibody, the titer of the antibody can also be measured with a commercially available analytical instrument such as Cedex Bio manufactured by F. Hoffmann-La Roche, Ltd.

Examples of the column that is used for affinity chromatography include a protein A column and a protein G column. Examples of the chromatography other than affinity chromatography include ion exchange chromatography, hydrophobic chromatography, gel filtration, reverse phase chromatography, and adsorption chromatography. The chromatography can be carried out using liquid phase chromatography such as high performance liquid chromatography (HPLC) or fast protein liquid chromatography (FPLC).

It is noted that it is also possible to modify the product or partially remove a peptide thereof by allowing an appropriate polypeptide modifying enzyme to act on the useful substance before or after purification. As the polypeptide modifying enzyme, for example, trypsin, chymotrypsin, lysyl endopeptidase, protein kinase, or glucosidase is used.

The product that is produced according to the present invention can be used, for example, for a biopharmaceutical product, or regenerative medicine.

The present invention will be more specifically described using the following examples; however, it is not limited by the examples.

Examples

<Establishment of antibody-producing cell>

A vector containing a nucleic acid sequence encoding IgG1 was constructed, and the constructed vector was introduced into CHO-DG44 cells, whereby CHO-DG44 cells expressing IgG1 (IgG1 cells) were produced. The construction of the vector and the introduction thereof into the cell were carried out according to Example 2 of JP2016-517691A. As described above, CHO cells producing monoclonal antibodies were prepared and used in the following experiment.

<Cell culture>

In Examples and Comparative Examples, experiments were carried out using the cell culture device shown in Fig. 1.

1.3 L of a culture medium was charged into a culture tank having a total capacity of 3 L (Biostat (registered trademark)) manufactured by Sartorius AG. CHO cells were seeded in the above culture medium at $0.5 \times 10^6$ cells/mL. At a stirring rotation speed of 160 rpm, 38 mL/min of air and 2 mL/min of $CO_2$ from the upper surface, and $O_2$ from the bottom surface were automatically controlled and supplied from a sparger installed in the culture tank so that the oxygen concentration in the culture solution was 80%.

After culturing for 2 days after seeding, the cell culture solution was continuously filtered with ATF2 manufactured by Repligen Corporation, and the recovered liquid was recovered while continuously supplying a culture medium at a perfusion ratio of 0.8 vvd.

In addition, on the 5th day, the perfusion ratio was further changed to 1.5 vvd.

Thereafter, in a case where the cell density reached $120 \times 10^6$ cells/mL, the cell bleeding was carried out while a part of the culture solution was drained so that the cell density was maintained at $120 \times 10^6$ cells/mL.

The culture conditions (the culture medium, the cell bleeding conditions, and the like) to be used in this case were set as shown in Table 1 to carry out Examples 1 to 6 and Comparative Examples 1 and 2. It is noted that each of culture media A to C shown in Table 1 is a culture medium obtained by adding glutamine and glucose to a commercially available culture medium so that the amounts thereof were an amount shown in Table 1.

Thereafter, the culture was continued for 10 days or more, and the evaluation described below was carried out.

In a case of the 50 L culture, the culture was carried out under the same conditions as those in the 1.3 L culture, except that 50 L of a culture medium was charged into a culture tank of Hypeforma 50 manufactured by Thermo Fisher Scientific Inc., the stirring rotation speed was set to 250 rpm, the setting was such that an upper surface air of 0.475 L/min and $CO_2$ of 0.025 L/min, and ATF4 manufactured by Repligen Corporation was used.

In a case of the 500 L culture, the culture was carried out under the same conditions as those in the 1.3 L culture, except that 500 L of a culture medium was charged into a culture tank of Hypeforma 500 manufactured by Thermo Fisher Scientific Inc., the stirring rotation speed was set to 150 rpm, the setting was such that an upper surface air of 4.75 L/min and $CO_2$ of 0.25 L/min, and ATF10 manufactured by Repligen Corporation was used.

<Automatic control method for cell bleeding>

In a case where the cell bleeding was automatically controlled, the cell bleeding was continuously carried out automatically using a FUTURA sensor manufactured by ABER Instruments Ltd. while monitoring the electrostatic capacity of the culture solution. Specifically, the measurement was carried out by the following method.

(1) An electrostatic capacity serving as a target is set.
(2) A weight control value of the culture tank is set.
(3) The electrostatic capacity of the culture solution is measured at a period of 0.1 s or less.
(4) A pump for draining the culture solution is driven at a rate of 0.3 vvd to 3 vvd in a case where the electrostatic capacity of the culture solution exceeds the target.
(5) A culture medium is automatically supplied into the culture tank while the weight of the culture tank is measured so that the liquid amount of the culture solution is roughly kept constant.
(6) The pump for draining the culture solution is stopped in a case where the electrostatic capacity of the culture solution is smaller than the target by 0.01% or more.

<Measurement and evaluation method>

(1) Measurement of viable cell density and average diameter and viability of viable cells

The culture solution in the culture tank was extracted and subjected to measurement using Cell Viability Analyzer Vi-cell XR manufactured by Beckman Coulter, Inc. It is noted that for the Vi-cell software, Vi-cell XR2.04 was used, and the parameters at the time of measurement were set as follows.

Min diameter: 6 $\mu$m
Max diameter: 50 $\mu$m
Dilution: In a case where the cell density was $100 \times 10^5$ cells/mL or less, the sample was not diluted, and the measurement was carried out at a Dilution of 1. In a case where the cell density was $100 \times 10^5$ cells/mL or more, the sample was diluted 10 times, and the measurement was carried out at a Dilution of 10.
Cell brightness: 85%
Cell sharpness: 100
Viable cell spot brightness: 75%
Viable cell spot area: 5%
Minimum circularity: 0
Decluster degree: Medium

The measurement was carried out once a day by the above method.

(2) Measurement of concentrations of Gln, Gluc, and $NH_4$

The culture solution was extracted, and the cells were filtered with Whatman (pore size: 0.2 $\mu$m, diameter: 25 mm) manufactured by Cytiva. Then, The filtrate was measured with a commercially available analysis apparatus such as Bioprofile FLEX2 manufactured by Nova Biomedical K.K.
The measurement was carried out once a day by the above method.

(3) Measurement of LDH

The culture solution was extracted, and the cells were filtered with Whatman (pore size: 0.2 $\mu$m, diameter: 25 mm) manufactured by Cytiva. Then, the filtrate was measured with Cedex Bio manufactured by F. Hoffmann-La Roche, Ltd.
The measurement was carried out once a day by the above method.

(4) Measurement of titer

The culture solution was extracted, and the cells were filtered with Whatman (pore size: 0.2 $\mu$m, diameter: 25 mm) manufactured by Cytiva. Then, the filtrate was measured with Cedex Bio manufactured by F. Hoffmann-La Roche, Ltd.
The measurement was carried out once a day by the above method.

(5) Measurement of mannose 5 (M5)

The evaluation of the mannose 5 of the antibody was carried out as an evaluation standard of Examples and Comparative Examples. The profile of the N-type sugar chain including mannose 5 was measured by the following method. The antibody in the antibody recovered liquid obtained was subjected to a digestion treatment with peptide-N-glycosidase F to cut out the N-type sugar chain. Thereafter, the cut sugar chain was subjected to fluorescence labeling with 2-aminopyridine. Then, the mannose 5 was subjected to quantitative analysis by reverse phase high performance liquid chromatography (HPLC) to calculate the peak of mannose 5 with respect to the total peak area.
A case where the content of the mannose 5 was less than 4% was evaluated as A, a case where the content thereof was 4% to 7% was evaluated as B, and a case where the content thereof exceeded 7% was evaluated as C.

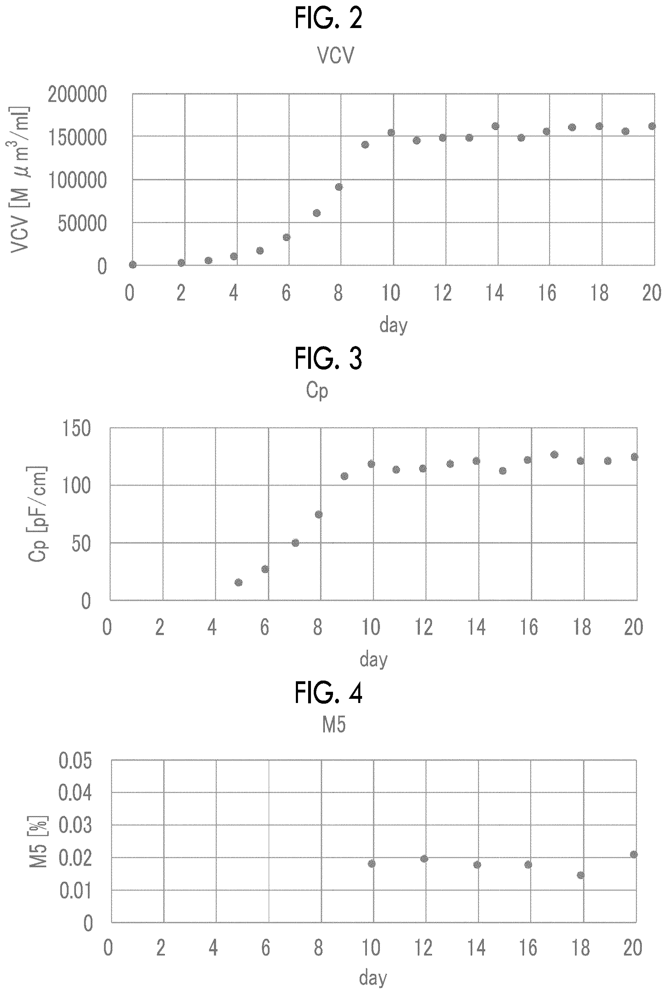

Fig. 2 to Fig 4 show results obtained by measuring the transition of the VCV, the electrostatic capacity data, and the mannose 5 ratio in Example 1.
Fig. 2 to Fig 3 show measurement results of each of Examples and each of Comparative Examples.

[Table 1]

| | Culture conditions | | | | | | | | | |
|---|---|---|---|---|---|---|---|---|---|---|
| | Amount of culture solution | Culture medium | | | Stirring | Dissolved oxygen concentration | Flow rate of gas from 20 μm sparger | | Cell bleeding | |
| | | Culture medium | Gln | Gluc | | | O₂ (Automatic control of dissolved oxygen) | Air (manual control) | Monitoring method | Control method |
| | | Kind | Concentration | Concentration | Rotation speed | | | | | |
| | [L] | | mmol/L | g/L | rpm | % | LPM | LPM | | |
| Example 1 | 1.3 | Culture medium A | 7 | 14 | 160 | 80 | 0.05 | 0 | Electrostatic capacity type in-line sensor | Automatic |
| Example 2 | 1.3 | Culture medium A | 7 | 14 | 160 | 80 | 0.05 | 0 | Absent | Manual (continuous treatment while finely adjusting pump setting every day) |
| Example 3 | 1.3 | Culture medium B | 10 | 17 | 160 | 80 | 0.05 | 0 | Electrostatic capacity type in-line sensor | Automatic |
| Example 4 | 1.3 | Culture medium A | 7 | 14 | 240 | 80 | 0.05 | 0 | Electrostatic capacity type in-line sensor | Automatic |
| Example 5 | 50 | Culture medium A | 7 | 14 | 250 | 80 | 3 | 0 | Electrostatic capacity type in-line sensor | Automatic |
| Example 6 | 500 | Culture medium A | 7 | 14 | 150 | 80 | 15 | 5 | Electrostatic capacity type in-line sensor | Automatic |

(continued)

| | Culture conditions | | | | | | | | | |
| | Amount of culture solution | Culture medium | | | Stirring | Dissolved oxygen concentration | Flow rate of gas from 20 μm sparger | | Cell bleeding | |
| | | Culture medium | Gln | Gluc | | | O$_2$ (Automatic control of dissolved oxygen) | Air (manual control) | Monitoring method | Control method |
| | | Kind | Concentration | Concentration | Rotation speed | | | | | |
| | [L] | | mmol/L | g/L | rpm | % | LPM | LPM | | |
| Comparative Example 1 | 1.3 | Culture medium C | 14 | 21 | 160 | 80 | 0.05 | 0 | Absent | Manual (once/day) |
| Comparative Example 2 | 1.3 | Culture medium C | 14 | 21 | 240 | 80 | 0.05 | 0.08 | Absent | Manual (once/day) |

[Table 2]

| | Data on electrostatic capacity during culture | | | | Measurement with Vicell | | | | | | | | | |
| | | | | | VCD | | | | Averaged diameter of cells | | VCV | | | |
| | Minimum | Maximum | Average | Variation | Minimum | Maximum | Average | Variation | Minimum | Maximum | Minimum | Maximum | Average | Variation |
| | pF/cm | | | ±% | $\times 10^6$ cells/mL, | | | ±% | $\mu$m | | $\times 10^6$ $\mu$m$^3$/mL | | | ±% |
| Example 1 | 112 | 126 | 119 | 6 | 110 | 129 | 120 | 8 | 1326 | 13.69 | 1.5E+05 | 1.6E+05 | 1.5E+05 | 6 |
| Example 2 | - | - | - | - | 109 | 130 | 119 | 9 | 1326 | 13.98 | 1.5E+05 | 1.7E+05 | 1.5E+05 | 12 |
| Example 3 | 111 | 125 | 120 | 8 | 110 | 129 | 120 | 8 | 13.4 | 14 | 1.5E+05 | 1.7E+05 | 1.6E+05 | 8 |
| Example 4 | 112 | 126 | 114 | 11 | 109 | 130 | 120 | 9 | 128 | 13.4 | 1.3E+05 | 1.6E+05 | 1.4E+05 | 16 |
| Example 5 | 108 | 127 | 115 | 10 | 106 | 127 | 119 | 11 | 13.2 | 13.8 | 1.3E+05 | 1.7E+05 | 1.5E+05 | 12 |
| Example 6 | 109 | 128 | 116 | 10 | 111 | 131 | 119 | 10 | 13.7 | 14 | 1.5E+05 | 1.9E+05 | 1.7E+05 | 12 |
| Comparative Example 1 | - | - | - | - | 107 | 144 | 119 | 21 | 13.2 | 14.1 | 1.4E+05 | 2.0E+05 | 1.6E+05 | 24 |
| Comparative Example 2 | - | - | - | - | 108 | 136 | 119 | 14 | 12.52 | 13.7 | 1.2E+05 | 1.8E+05 | 1.5E+05 | 26 |

[Table 3]

| | Measurement with FLEX2 | | | | | | Measurement with Cedex | | | | | HPLC | Evaluation |
|---|---|---|---|---|---|---|---|---|---|---|---|---|---|
| | Gln-C | | Gluc-C | | NH4-C | | LDH | | LDH/VCD | | Titer | Mannose 5 | |
| | Minimum i | Maximum | Minimum | Maximum | Minimum | Maximum | Minimum | Maximum | Minimum | Maximum | Average | | |
| | mmol/L | | g/L | | mmol/L | | U/L | | | | g/L | Average % | |
| Example 1 | 0.3 | 1.0 | 3.4 | 4.2 | 2.8 | 3.9 | 500 | 2200 | 4 | 18 | 14 | 1.8 | A |
| Example 2 | 0.3 | 0.9 | 2.6 | 4.0 | 2.8 | 4.3 | 600 | 1700 | 5 | 14 | 14 | 3.1 | A |
| Example 3 | 14 | 2.2 | 4.9 | 6.7 | 4.1 | 6.2 | 600 | 2300 | 5 | 19 | 1.3 | 5.3 | B |
| Example 4 | 0.2 | 0.9 | 3.1 | 4.0 | 3.2 | 4.8 | 1300 | 4900 | 11 | 41 | 1.2 | 41 | B |
| Example 5 | 0.5 | 1.5 | 3.1 | 4.9 | 3.5 | 43 | 500 | 1700 | 4 | 14 | 14 | 3.2 | A |
| Example 6 | 0.3 | 0.6 | 2.1 | 3.7 | 3.9 | 4.5 | 700 | 2200 | 6 | 18 | 1.6 | 3.7 | A |
| Comparative Example 1 | 2.6 | 3.1 | 8.2 | 8.8 | 5.3 | 8.2 | 900 | 3200 | 8 | 27 | 1.1 | 8.3 | C |
| Comparative Example 2 | 33 | 4.1 | 9.0 | 9.5 | 6.6 | 91 | 2200 | 6700 | 18 | 56 | 1.1 | 9.5 | C |

Explanation of References

1: sparger air supply pipe

2: air supply pipe

3: culture medium supply pipe

4: gas exhaust pipe

5: gas exhaust filter

6: sampling tube

7: bleeding tube

8: electrostatic capacity sensor

9: dissolved oxygen sensor

10: culture container

11: sparger

12: stirring blade

13: perfusion device

14: arrow

15: culture medium supply pump

16: delivery pump

**Claims**

1. A production method for a product, comprising:

    culturing cells in a culture container,
    wherein the culturing is a perfusion culture method,
    Expressions (1) to (5) are satisfied in a production period of a product after reaching a target viable cell density,
    a variation of VCD during the production period is ±20% or less with respect to an average viable cell density during the production period, and
    a variation of VCV during the production period is ±20% or less with respect to an average value of the VCV during the production period,

$$0 \leq \text{Gln-C} \leq 2.5 \qquad \text{Expression (1)}$$

$$0.5 \leq \text{Gluc-C} \leq 8.0 \qquad \text{Expression (2)}$$

$$1.0 \leq \text{NH4-C} \leq 8.0 \qquad \text{Expression (3)}$$

$$50 \leq VCD \leq 300 \qquad \text{Expression (4)}$$

$$0.6 \times 10^5 \leq VCV \leq 4.0 \times 10^5 \qquad \text{Expression (5)}$$

in the expressions, Gln-C indicates a glutamine concentration [mmol/L] in a culture solution, Gluc-C indicates a glucose concentration [g/L] in the culture solution, NH4-C indicates an ammonia concentration [mmol/L] in the culture solution, VCD indicates a viable cell density [$\times 10^6$ cells/mL], VCV indicates VCD $\times \pi/6 \times Cd^3$ [$\times 10^6$ $\mu m^3$/mL], and Cd indicates an average diameter [$\mu m$] of viable cells.

2. The production method for a product according to claim 1,
   wherein the culture solution is bled such that the variation of VCD during the production period is $\pm 20\%$ or less with respect to the average viable cell density during the production period.

3. The production method for a product according to claim 2,
   wherein in the bleeding, the culture solution is bled while an electrostatic capacity of the culture solution is subjected to in-line measurement.

4. The production method for a product according to claim 3,
   wherein the bleeding is automatically controlled.

5. The production method for a product according to claim 3 or 4,
   wherein a variation of the electrostatic capacity of the culture solution, where the electrostatic capacity is subjected to in-line measurement, is $\pm 20\%$ or less with respect to an average value of an electrostatic capacity during the production period.

6. The production method for a product according to any one of claims 3 to 5,
   wherein the bleeding includes:

   (i) setting an electrostatic capacity serving as a target;
   (ii) setting a weight control value of the culture container;
   (iii) measuring the electrostatic capacity of the culture solution at a period of 0.1 seconds or less;
   (iv) driving a pump for draining the culture solution at a rate of 0.3 vvd to 3 vvd in a case where the electrostatic capacity of the culture solution exceeds the target (here, vvd means "volume of drained culture solution/volume of culture solution/day"); and
   (v) automatically supplying a culture medium to the culture container while measuring a mass of the culture solution such that a variation of a liquid amount of the culture solution is within 10%; and
   (vi) stopping the pump for draining the culture solution in a case where the electrostatic capacity of the culture solution is smaller than the target by 0.01% or more.

7. The production method for a product according to any one of claims 1 to 6,
   wherein Expression (6) is satisfied in the production period,

$$2 \leq LDH/VCD \leq 40 \qquad \text{Expression (6)}$$

in the expression, LDH indicates a concentration [U/L] of lactate dehydrogenase in the culture solution, and VCD indicates a viable cell density [$\times 10^6$ cells/mL],

8. The production method for a product according to any one of claims 1 to 7,
   wherein the cell is an animal cell.

9. The production method for a product according to any one of claims 1 to 8,
   wherein the cell is a cell that produces a protein.

10. The production method for a product according to any one of claims 1 to 9,
    wherein the cell is a cell that produces an antibody.

**11.** The production method for a product according to any one of claims 1 to 10,
wherein the cell is a CHO cell.

**12.** A product that is produced by the production method for a product according to any one of claims 1 to 11.

FIG. 1

## FIG. 2

VCV

## FIG. 3

Cp

## FIG. 4

M5

## INTERNATIONAL SEARCH REPORT

International application No.

**PCT/JP2023/012126**

### A. CLASSIFICATION OF SUBJECT MATTER

*C12P 21/08*(2006.01)i; *C07K 16/18*(2006.01)i; *C12N 5/071*(2010.01)i; *C12N 5/10*(2006.01)i
FI: C12P21/08; C07K16/18; C12N5/071; C12N5/10

According to International Patent Classification (IPC) or to both national classification and IPC

### B. FIELDS SEARCHED

Minimum documentation searched (classification system followed by classification symbols)

C12P21/08; C07K16/18; C12N5/071; C12N5/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | JP 2020-005511 A (HITACHI LTD) 16 January 2020 (2020-01-16) <br> claims 1-10, paragraphs [0017], [0031], [0034], [0036], [0040]-[0055], [0091]-[0094], fig. 2-3, 5, table 2 | 12 |
| Y | | 1-12 |
| Y | DOWNEY, B. J. et al. A novel approach for using dielectric spectroscopy to predict viable cell volume (VCV) in early process development. BIOTECHNOL. PROG. 2014, vol. 30, no. 2, pp. 479-487, DOI: 10.1002/btpr.1845, Published online 19 December 2013 <br> abstract, p. 481, left column, line 28 to p. 481, right column, line 10 | 1-12 |
| Y | JP 2020-533983 A (BRISTOL-MYERS SQUIBB COMPANY) 26 November 2020 (2020-11-26) <br> claims 1-29 | 1-12 |

☐ Further documents are listed in the continuation of Box C.      ☑ See patent family annex.

| | |
|---|---|
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **01 June 2023** | **13 June 2023** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

**INTERNATIONAL SEARCH REPORT**
Information on patent family members

International application No.

**PCT/JP2023/012126**

| Patent document cited in search report | | | Publication date (day/month/year) | Patent family member(s) | | | Publication date (day/month/year) |
|---|---|---|---|---|---|---|---|
| JP | 2020-005511 | A | 16 January 2020 | (Family: none) | | | |
| JP | 2020-533983 | A | 26 November 2020 | US | 2020/0255785 | A1 | |
| | | | | claims 1-29 | | | |
| | | | | WO | 2019/055796 | A1 | |
| | | | | EP | 3681989 | A1 | |
| | | | | KR | 10-2020-0047702 | A | |
| | | | | CN | 111868223 | A | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021505182 A
- JP 2020033364 A
- JP 2020533983 A
- JP 2016517691 A